(19) Europäisches Patentamt
European Patent Office
Office européen des brevets

(11) EP 3 026 038 A1

(12) EUROPEAN PATENT APPLICATION

(43) Date of publication:
**01.06.2016 Bulletin 2016/22**

(21) Application number: **14195176.4**

(22) Date of filing: **27.11.2014**

(51) Int Cl.:
***C07C 67/08*** (2006.01) ***C01G 25/02*** (2006.01)
***B01J 23/00*** (2006.01) ***B01J 23/10*** (2006.01)
***B01J 35/00*** (2006.01) ***B01J 37/02*** (2006.01)
*B01J 35/02* (2006.01) *B01J 35/10* (2006.01)
*B01J 37/03* (2006.01) *B01J 37/06* (2006.01)
*B01J 37/08* (2006.01) *B01J 37/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Evonik Degussa GmbH**
**45128 Essen (DE)**

(72) Inventors:
- **Wieland, Stefan**
**63456 Hanau (DE)**
- **Lansink Rotgerink, Hermanus Gerhardus Jozef**
**63776 Mömbris/Mensengesäß (DE)**
- **Trionfetti, Cristiano**
**60528 Frankfurt (DE)**

(54) **Zirconium and yttrium mixed oxide catalyst for alcoholysis reactions**

(57) The present invention relates to a mixed oxide containing zirconium and yttrium, wherein the tetragonal crystalline phase is present in a range of from 85 % to 100 % and wherein the content of Na is in a range of from 20 ppm to 150 ppm. Furthermore the present invention relates to a process for the production of said mixed oxide and its use as a catalyst or catalyst system.

EP 3 026 038 A1

## Description

The present invention relates to a mixed oxide containing zirconium and yttrium, wherein the tetragonal crystalline phase is present in a range of from 85 % to 100 % and wherein the content of Na is in a range of from 20 ppm to 150 ppm. Furthermore the present invention relates to a process for the production of said mixed oxide and its use as a catalyst or catalyst system.

Carboxylic acid esters are industrially important compounds. These carboxylic acid esters can be produced from carboxylic acid amides: for example, methyl acetate is produced from acetic acid amides; methyl methacrylate from methacrylic acid amide; methyl acrylate from acrylic acid amide; or methyl $\alpha$-hydroxyisobutyrate from $\alpha$-hydroxyisobutyric acid amide. For producing carboxylic acid esters from carboxylic acid amides, several methods of reacting a carboxylic acid amide with an alcohol in the presence of a catalyst are known. In particular solid acid catalysts e.g., silica, alumina, zeolites or silico-aluminate, are reported to be effective for the production of carboxylic acid esters.

EP2415750 (Mitsubishi Gas Chemical Company) discloses a process for producing $\alpha$-hydroxycarboxylic esters from $\alpha$-hydroxycarboxylic amides and aliphatic alcohols in the gas phase in the presence of a zirconium dioxide catalyst. The zirconium dioxide catalyst may contain at least one element selected from the $2^{nd}$ to $4^{th}$ groups, $7^{th}$ group and $9^{th}$ to $13^{th}$ groups in the periodic table, lanthanoids, antimony and bismuth. The catalyst may be obtained by a precipitation method, an impregnation method or co-precipitation method. There is no information about crystalline phases or Na content and the influence of these parameters on the alcoholysis reaction in this document.

JPH06345692 (Nitto Chem. Ind. Co. Ltd.) discloses a process for producing $\alpha$-hydroxyisobutyric acid ester from $\alpha$-hydroxyisobutyric acid amide and an alcohol in the liquid phase in the presence of an insoluble solid acid catalyst. This catalyst can be chosen from oxides of one or more elements selected from the group consisting of Sb, Sc, Y, La, Ce, Ti, Zr, Hf, V, Nb, Ta, Cr, Mo, W, Tc, Re, Fe, Co, Ni, Cu, Al, Si, Sn, Pb, and Bi. There is no information about crystalline phases or Na content and the influence of these parameters on the alcoholysis reaction in this document.

The above-mentioned catalysts are either suitable for gas phase or for liquid phase reactions. They do not show a good performance regarding selectivity and yield in both kinds of alcoholysis reaction. Furthermore, there is still a need for catalysts with improved characteristics with regard to activity and selectivity in alcoholysis reactions, especially for versatile catalysts that show a good performance in gas phase and liquid phase reactions.

Therefore, the object of the present invention is to provide an improved catalyst for alcoholysis reactions, especially for producing carboxylic acid esters from carboxylic acid amides through direct reaction with alcohols, which shows a good performance regarding selectivity and yield in gas phase as well as in liquid phase reactions.

This object is solved by a mixed oxide containing zirconium and yttrium, wherein the tetragonal crystalline phase is present in an amount of from 85 % to 100 % and wherein the content of Na is in a range of from 20 ppm to 150 ppm.

Furthermore, the present invention provides a mixed oxide containing zirconium and yttrium, wherein the tetragonal crystalline phase is present in an amount of from 85 % to 100 % and wherein the content of Na is in a range of from 20 ppm to 150 ppm and wherein the molar ratio of Zr:Y, calculated as $ZrO_2$ and $Y_2O_3$, is in a range of from 90:10 to 98:2.

A mixed oxide in the sense of the present invention defines an oxide that contains cations of more than one chemical element. In the present invention these cations are at least zirconium and yttrium. The mixed oxide is not a physical mixture of two oxides but a chemical mixture with an own crystal structure.

The molar ratio of Zr:Y in the mixed oxide, calculated as $ZrO_2$ and $Y_2O_3$, is in a range of from 90:10 to 98:2, preferably 95:5 to 98:2, more preferably 96:4.

The tetragonal crystalline phase is present in an amount in a range of from 85 % to 100 %, preferably in a range of from 87 % to 98 %. The tetragonal crystalline phase of the mixed oxide is referred to entry [01-070-4428] of the ICDD Database (International Centre for Diffraction Data).

The amount of tetragonal crystalline phase is determined using the relationship between the scaling factors and the weight fraction of the phases after refinement of the X-Ray Powder Diffractogram using the Rietveld method according to Acta Cryst. 1967, 22, 151-152 and J. Appl. Cryst. 1969, 2, 65-71.

The mixed oxide can have any form or shape, e.g. powder, particles, granules, spheres, shaped bodies like cylindrical extrudates and cut cylindrical extrudates, tablets, rings, pentarings, or trilobes. Preferably the mixed oxide is in the form of rod-shaped granules.

In order to transform the mixed oxide into these shapes, any moulding technique or apparatus known to the skilled person can be applied. To obtain granules, any granulation technique and apparatus known to the skilled person can be applied, e.g. spray dryer, high shear granulator, twin screw granulator, fluidized bed granulator, rotating plate or Eirich intensive mixer. Preferably, granulation is performed in an Eirich intensive mixer type R to yield rod-shaped granules.

Said mixed oxide granules may have an average particle size in the range of from 0.1 mm to 10 mm. Preferably, the granules have an average particle size in the range of from 1 mm to 5 mm. After granulation the particles may be fractionated to obtain defined particle sizes. Fractionation in the sense of the present invention is defined as a sieve analysis with a mesh sieving machine, wherein the size of the slots of the mesh is selected in such a way that particles

with a size in the range of from 0.1 mm to 10 mm (mesh 1), 0.8 mm to 5.0 mm (mesh 2), 0.8 mm to 2.5 mm (mesh 3) or 1.0 mm to 2.8 mm (mesh 4) are collected.

The bulk density of said mixed oxide granules is in a range of from 1100 g/L to 1200 g/L, preferably 1150 g/L.

The BET surface area of the mixed oxide granules is in a range of from 100 $m^2/g$ to 200 $m^2/g$, preferably 110 $m^2/g$ to 140 $m^2/g$, more preferably 120 $m^2/g$ to 130 $m^2/g$.

BET surface area is determined in accordance with DIN 66131 and DIN ISO 9277.

The abrasion of said mixed oxide granules is in a range of from 2.5 wt-% to 6.0 wt-%, preferably 4.0 wt-% to 6.0 wt-%.

Abrasion is determined in accordance with ASTM D4058-96.

Furthermore, the present invention provides a process for the production of a mixed oxide containing zirconium and yttrium, wherein the process comprises the following steps:

a. providing zirconium hydroxide with a content of Na in a range of from 20 ppm to 150 ppm,

b. providing an aqueous solution containing at least one yttrium salt, wherein the volume of said solution corresponds to 40 % to 75 % of the pore volume $P_v(H_2O)$ of the zirconium hydroxide, and wherein yttrium is present in said solution in such an amount that the mixed oxide exhibits 2 wt-% to 10 wt-% yttrium, calculated as $Y_2O_3$ and referring to the total weight of the mixed oxide,

c. contacting zirconium hydroxide with the solution obtained in step b),

d. adding water in an amount of from 0 % to 35 % of the pore volume $P_v(H_2O)$ of zirconium hydroxide, wherein the total amount of liquid - in the form of aqueous yttrium salt solution and water - added to the zirconium hydroxide is at most 75 % of the pore volume $P_v(H_2O)$ of the zirconium hydroxide,

e. drying the zirconium hydroxide,

f. calcining the zirconium hydroxide to obtain the mixed oxide.

Zirconium hydroxide with a Na content in the range of from 20 ppm to 150 ppm can be prepared by any precipitation techniques known to the skilled artisan, alternatively commercially available zirconium hydroxide is used. Any commercially available zirconium hydroxide can be used for the present invention, wherein typical examples are zirconium hydroxides from MEL, e.g. XZ01501/03, XZ01247/01 and XZ01501/09. Preferably, the zirconium hydroxide exhibits special quality characteristics regarding pore volume, LOD, LOI, pH, pI and particle size distribution.

Preferably, the pore volume ($P_v(H_2O)$) of the zirconium hydroxide is in the range of from 0.5 ml/g to 1.0 ml/g, preferably 0.54 ml/g to 0.7 ml/g.

Preferably, the LOD (loss on drying, at 130 °C for 4h) of the zirconium hydroxide is in a range of from 11.0 wt-% to 13.5 wt-%, preferably 12.5 wt-% to 13.5 wt-%.

Preferably, the LOI (loss on ignition, at 450 °C for 2h) of the zirconium hydroxide is in a range of 20.0 wt-% to 25.0 wt-%, preferably 22.5 wt-% to 24.0 wt-%.

Preferably, the isoelectic point, referred to as pI and sometimes abbreviated to IEP, of the zirconium hydroxide is in the range of pH = 8.0 - 8.2

Preferably, the pH value of the zirconium hydroxide is in a range of from pH = 6.0 - 6.5.

The content of Na in the zirconium hydroxide as well as the mixed oxide is in a range of from 20 ppm to 150 ppm, more preferably the Na content is in a range of from 80 ppm to 100 ppm.

The content of Cl in the zirconium hydroxide as well as the mixed oxide is <100 ppm, preferably the Cl content is in a range of from 100 ppm to 5 ppm, more preferably the Cl content is in a range of from 40 ppm to 5 ppm.

To achieve the desired Na content the zirconium hydroxide can be thoroughly washed with $NH_4NO_3$ before use.

For the production of the mixed oxide of the present invention the zirconium hydroxide can have any form or shape, e.g. powder or any kind of particles. Preferably, the zirconium hydroxide is in the form of particles. The particle size distribution of said zirconium hydroxide particles is measured by laser diffraction techniques. The most common approach for expressing results from laser diffraction is to report the D10, D50 and D90 values based on a volume distribution, wherein the volume of the particles is plotted versus percentage.

The D10 value is defined as the particle size, i.e. diameter, in mm where 10 % of the particles are below this diameter. The D50 value is defined as the particle size, i.e. diameter, in mm where 50 % of the particles are below this diameter. This value is also called median particle size. The D90 value is defined as the particle size, i.e. diameter in mm where 90 % of the particles are below this diameter. Figure 1 illustrates this. The SPAN calculation is the most common format to express distribution width. The SPAN is defined according to the following formula:

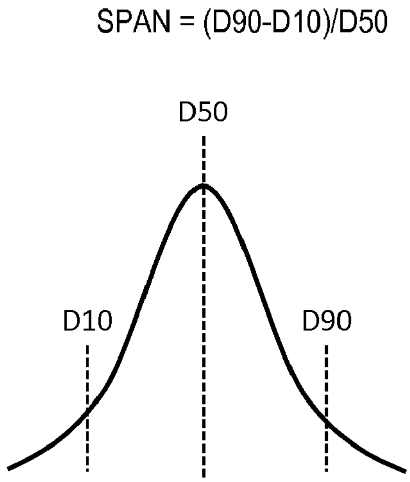


Figure 1: Particle size distribution curve.

In one embodiment of the present invention, the zirconium hydroxide is in the form of a powder. In another embodiment of the present invention, the zirconium hydroxide is in the form of particles which exhibit the following particle size distribution, wherein the particle size distribution is determined by laser diffraction: D10 = 0.8 - 3.0 μm, D50 = 2.0 - 7.0 μm, D90 = 5.0 - 25.0 μm, SPAN = 1.0 - 5.0, preferably D10 = 0.8 - 1.3 μm, D50 = 2.0 - 5.1 μm, D90 = 5.0 - 23.0 μm, SPAN = 1.3-3.5.

In a special embodiment of the present invention, the zirconium hydroxide is in the form of particles which exhibit the quality characteristics as shown in Table 1 entry 1. More preferably, the zirconium hydroxide is in the form of particles which exhibit the quality characteristics as shown in Table 1 entry 2.

Table 1: Quality characteristics of preferred zirconium hydroxide particles.

| Zirconium hydroxide | Na content [ppm] | BET [$m^2/g$] | Porevolume Pv($H_2O$) [ml/g] | pH | pI | LOD, LOI [wt-%] | D10/D50/D90 [μm], SPAN |
|---|---|---|---|---|---|---|---|
| 1 | 20-150 | 300-400 | 0.5-1.0 | 6.0-6.5 | 8.0-8.2 | LOD = 11.0-13.5 LOI = 20.0-25.0 | D10 = 0.8-3.0, D50 = 2.0-7.0, D90 = 5.0-25.0, SPAN = 1.0-5.0 |
| 2 | 80-100 | 330-350 | 0.54-0.7 | 6.0-6.5 | 8.0-8.2 | LOD = 12.5-13.5 LOI = 22.5-24.0 | D10 = 0.8-1.3, D50 = 2.0-5.1, D90 = 5.0-23.0, SPAN = 1.3-3.5 |

For the production of the mixed oxide according to the present invention the yttrium salt is not critical and it may be any commercially available yttrium salt. Preferably, the yttrium salt is selected from the group consisting of nitrate, chloride, iodide, fluoride, acetate, hydroxide, oxalate, trifluoroacetate, acetylacetonate, trifluoromethanesulfonate, isopropoxide, butoxide, phosphate, or mixtures thereof. More preferably, the yttrium salt is yttrium nitrate.

Contacting under agitation in the sense of the present invention may be any technique known to the skilled person. Preferably, contacting under agitation is a technique, which is selected from spraying under agitation, impregnating under agitation, and kneading. More preferably, contacting means impregnating under agitation, especially pore volume impregnation. Any suitable method of agitation can be applied, e.g. stirring, shaking, or mixing. Preferably, agitation means stirring constantly with a moderate rotational speed.

Drying is performed at a suitable temperature. Preferably, drying is performed at a temperature in the range of from 70 °C to 150 °C for 2h-6h. More preferably, drying is performed at a temperature in the range of from 80 °C to 140 °C for 2h-6h, even more preferably drying is performed at 120 °C for 2h. Drying is preferably performed in a muffle furnace or conveyor dryer.

Calcining is performed at a suitable temperature. Preferably, calcining is performed at a temperature in the range of from 200 °C to 500 °C for 2h-4h. More preferably, calcining is performed at 450 °C for 2h. Calcining is preferably performed in a stationary oven or rotary kiln.

In a special embodiment of the process for the production of a mixed oxide containing zirconium and yttrium, the zirconium hydroxide is further treated by moulding before or after drying.

Furthermore, the present invention provides a process for the production of a mixed oxide containing zirconium and yttrium, wherein the process comprises the following steps:

a. providing zirconium hydroxide with a content of Na in a range of from 20 ppm to 150 ppm,

b. providing an aqueous solution containing at least one yttrium salt, wherein the volume of said solution corresponds to 40 % to 75 % of the pore volume $P_v(H_2O)$ of the zirconium hydroxide, and wherein yttrium is present in said solution in such an amount that the mixed oxide exhibits 2 wt-% to 10 wt-% yttrium, calculated as $Y_2O_3$ and referring to the total weight of the mixed oxide,

c. contacting zirconium hydroxide with the solution obtained in step b) under agitation,

d. adding water in an amount of from 0 % to 35 % of the pore volume $P_v(H_2O)$ of zirconium hydroxide, wherein the total amount of liquid - in the form of aqueous yttrium salt solution and water- added to the zirconium hydroxide is at most 75 % of the pore volume $P_v(H_2O)$ of the zirconium hydroxide,

e. drying the zirconium hydroxide at a temperature in the range of from 70 °C to 150 °C for 2h-6h,

f. calcining the zirconium hydroxide at a temperature in the range of from 200 °C to 500 °C for 2h-4h to obtain the mixed oxide.

Furthermore, the present invention provides a process for the production of a mixed oxide containing zirconium and yttrium, wherein the process comprises the following steps:

a. providing zirconium hydroxide with a content of Na in a range of from 20 ppm to 150 ppm,

b. providing an aqueous solution containing at least one yttrium salt, wherein the volume of said solution corresponds to 40 % to 75 % of the pore volume $P_v(H_2O)$ of the zirconium hydroxide, and wherein yttrium is present in said solution in such an amount that the mixed oxide exhibits 2 wt-% to 10 wt-% yttrium, calculated as $Y_2O_3$ and referring to the total weight of the mixed oxide,

c. contacting zirconium hydroxide with the solution obtained in step b),

d. adding water in an amount of from 0 % to 35 % of the pore volume $P_v(H_2O)$ of zirconium hydroxide, wherein the total amount of liquid - in the form of aqueous yttrium salt solution and water- added to the zirconium hydroxide is at most 75 % of the pore volume $P_v(H_2O)$ of the zirconium hydroxide,

e. drying the zirconium hydroxide at a temperature of 120 °C for 2h,

f. calcining the zirconium hydroxide at a temperature of 450 °C for 2h to obtain the mixed oxide.

Furthermore, the present invention provides a mixed oxide obtainable by a process comprising the following steps:

a. providing zirconium hydroxide with a content of Na in a range of from 20 ppm to 150 ppm,

b. providing an aqueous solution containing at least one yttrium salt, wherein the volume of said solution corresponds to 40 % to 75 % of the pore volume $P_v(H_2O)$ of the zirconium hydroxide, and wherein yttrium is present in said solution in such an amount that the mixed oxide exhibits 2 wt-% to 10 wt-% yttrium, calculated as $Y_2O_3$ and referring to the total weight of the mixed oxide,

c. contacting zirconium hydroxide with the solution obtained in step b),

d. adding water in an amount of from 0 % to 35 % of the pore volume $P_V(H_2O)$ of zirconium hydroxide, wherein the total amount of liquid - in the form of aqueous yttrium salt solution and water- added to the zirconium hydroxide is at most 75 % of the pore volume $P_V(H_2O)$ of the zirconium hydroxide,

e. drying the zirconium hydroxide,

f. calcining the zirconium hydroxide to obtain the mixed oxide.

Furthermore, the present invention provides a catalyst system comprising a mixed oxide containing zirconium and yttrium, wherein the tetragonal crystalline phase is present in an amount of from 85 % to 100 % and wherein the content of Na is in a range of from 20 ppm to 150 ppm, and a catalyst support. The catalyst support is not critical and may be selected from any suitable support material known to a skilled person. Preferably the support is selected from the group consisting of silica, alumina, titania, zirconia, magnesia, iron oxide, and mixtures thereof.

The mixed oxide of the present invention may be used as a catalyst or catalyst system. This catalyst or catalyst system may be used in alcoholysis reactions, preferably in methanolysis reactions for the production of carboxylic acid esters.

Furthermore, the present invention provides a process for an alcoholysis reaction for the production of carboxylic acid esters, wherein a mixed oxide containing zirconium and yttrium, wherein the tetragonal crystalline phase is present in a range of from 85 % to 100 % and wherein the content of Na is in a range of from 20 ppm to 150 ppm, is applied.

Furthermore, the present invention provides a process for an alcoholysis reaction for the production of carboxylic acid esters, wherein a mixed oxide containing zirconium and yttrium, wherein the tetragonal crystalline phase is present in a range of from 85 % to 100 % and wherein the content of Na is in a range of from 20 ppm to 150 ppm, applied and wherein the process is a methanolysis reaction.

Furthermore, the present invention provides a process for an alcoholysis reaction for the production of carboxylic acid esters, wherein a mixed oxide containing zirconium and yttrium, wherein the tetragonal crystalline phase is present in a range of from 85 % to 100 % and wherein the content of Na is in a range of from 20 ppm to 150 ppm, is applied and wherein the process is a methanolysis reaction that is performed as gas phase reaction.

Furthermore, the present invention provides a process for an alcoholysis reaction for the production of carboxylic acid esters, wherein a mixed oxide containing zirconium and yttrium, wherein the tetragonal crystalline phase is present in a range of from 85 % to 100 % and wherein the content of Na is in a range of from 20 ppm to 150 ppm, is applied and wherein the process is a methanolysis reaction that is performed as liquid phase reaction.

Furthermore, the present invention provides a process for the production of carboxylic acid esters, wherein a mixed oxide containing zirconium and yttrium, wherein the tetragonal crystalline phase is present in a range of from 85 % to 100 % and wherein the content of Na is in a range of from 20 ppm to 150 ppm, is applied and wherein the process is a methanolysis reaction that is performed as gas phase and the carboxylic acid ester is a hydroxyl carboxylic acid ester.

In the sense of the present invention, alcoholysis reactions for the production of carboxylic acid esters are defined as reaction of a carboxylic acid amide with an aliphatic alcohol ROH to obtain a carboxylic acid ester according to the following equation:

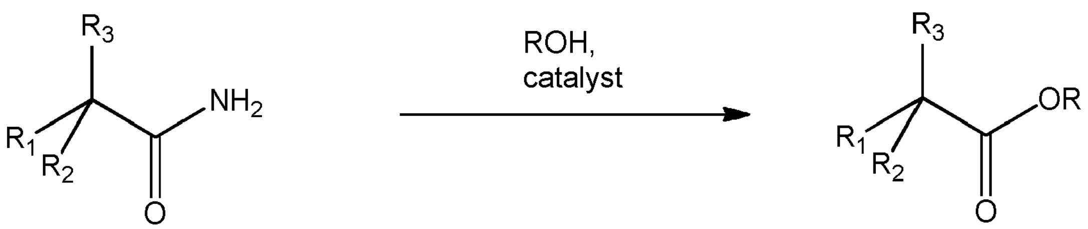


,

wherein
R is selected from the group consisting of $(C_1\text{-}C_{20})$-alkyl group, $(C_2\text{-}C_{20})$-alkenyl group, and $(C_3\text{-}C_{20})$-cycloalkyl group;
$R_1$ is selected from the group consisting of H, $(C_1\text{-}C_{20})$-alkyl group, $(C_2\text{-}C_{20})$-alkenyl group, $(C_3\text{-}C_{20})$-cycloalkyl group;
$R_2$ is selected from the group consisting of H, $(C_1\text{-}C_{20})$-alkyl group, $(C_2\text{-}C_{20})$-alkenyl group, $(C_3\text{-}C_{20})$-cycloalkyl group,; and
$R_3$ is selected from the group consisting of H, OH, $(C_1\text{-}C_{20})$-alkyl group, $(C_2\text{-}C_{20})$-alkenyl group, $(C_3\text{-}C_{20})$-cycloalkyl group.

A carboxylic acid amide in the sense of the present invention is defined as a compound of the following formula

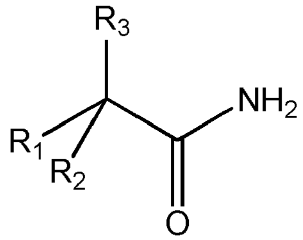

,

wherein

$R_1$ is selected from the group consisting of H, ($C_1$-$C_{20}$)-alkyl group, ($C_2$-$C_{20}$)-alkenyl group, ($C_3$-$C_{20}$)-cycloalkyl group,
$R_2$ is selected from the group consisting of H, ($C_1$-$C_{20}$)-alkyl group, ($C_2$-$C_{20}$)-alkenyl group, ($C_3$-$C_{20}$)-cycloalkyl group,
$R_3$ is selected from the group consisting of H, OH, ($C_1$-$C_{20}$)-alkyl group, ($C_2$-$C_{20}$)-alkenyl group, ($C_3$-$C_{20}$)-cycloalkyl group.

A carboxylic acid ester in the sense of the present invention is defined as a compound of the following formula

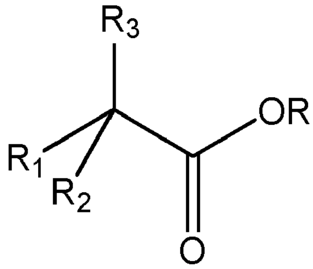

,

wherein

$R_1$ is selected from the group consisting of H, ($C_1$-$C_{20}$)-alkyl group, ($C_2$-$C_{20}$)-alkenyl group, ($C_3$-$C_{20}$)-cycloalkyl group,
$R_2$ is selected from the group consisting of H, ($C_1$-$C_{20}$)-alkyl group, ($C_2$-$C_{20}$)-alkenyl group, ($C_3$-$C_{20}$)-cycloalkyl group,
$R_3$ is selected from the group consisting of H, OH, ($C_1$-$C_{20}$)-alkyl group, ($C_2$-$C_{20}$)-alkenyl group, ($C_3$-$C_{20}$)-cycloalkyl group.

An aliphatic alcohol in the sense of the present invention is defined as a compound of the formula ROH, wherein R is selected from the group consisting of ($C_1$-$C_{20}$)-alkyl group, ($C_2$-$C_{20}$)-alkenyl group, and ($C_3$-$C_{20}$)-cycloalkyl group.

According to the present invention a ($C_1$-$C_n$)-alkyl group is a linear or branched alkyl group, wherein the sum of the carbon atoms is 1 to n. Preferred are linear ($C_1$-$C_{20}$)-alkyl groups, e.g. methyl, ethyl, n-propyl, n-butyl, n-pentyl, n-hexyl, n-heptyl, n-octyl, nonyl, n-decyl, n-undecyl, n-dodecyl, n-tetradecyl, n-pentadecyl, n-hexydecyl, n-heptadecyl, n-octadecyl, n-nonadecyl, n-icosyl.

According to the present invention a ($C_2$-$C_n$)-alkenyl group is a linear or branched alkyl group, wherein the sum of the carbon atoms is 2 to n and wherein at least one C-C double bond is present. Preferred are linear ($C_1$-$C_{20}$)-alkenyl groups, e.g. ethenyl, n-propenyl, n-butenyl, n-pentenyl, n-hexenyl, n-heptenyl, n-octenyl, nonenyl, n-decenyl, n-undecenyl, n-dodecenyl, n-tetradecenyl, n-pentadecenyl, n-hexydecenyl, n-heptadecenyl, n-octadecenyl, n-nonadecenyl, n-icosenyl.

According to the present invention a ($C_3$-$C_n$)-cycloalkyl group is a cyclic alkyl group with 3 to n carbon atoms, wherein mono-, bi- and tri-cyclic alkyl groups are included. Preferred are monocyclic ($C_3$-$C_{10}$)-cycloalkyl groups, e.g. cyclopropyl, cyclobutyl, cyclopentyl, cyclohexyl, cycloheptyl, cyclooctyl, cyclononyl, and cyclodecyl.

Preferably R is a ($C_1$-$C_{10}$)-alkyl group, more preferably R is a ($C_1$-$C_5$)-alkyl groups, most preferably R is selected from methyl and ethyl.

Preferred aliphatic alcohols are methanol, ethanol, propanol, n-butanol, 2-methyl-1-propanol, pentanol, hexanol, heptanol, 2-ethylhexanol, octanol, nonanol, decanol. More preferred are methanol and ethanol, wherein methanol is even more preferred.

Preferably, $R_1$ is selected from the group consisting of H, Me, and Et, wherein Me is most preferred.

Preferably, $R_2$ is selected from H, Me, and Et, wherein Me is most preferred.

Preferably, $R_3$ is selected from H, OH, Me, and Et, wherein OH is most preferred.

In a special embodiment, $R_1$ and $R_2$ are both Me, $R_3$ is OH and R is Me. In a further special embodiment, $R_1$ and $R_2$ are both Me, $R_3$ is OH and R is Et.

The mixed oxide of the present invention can be applied in the alcoholysos reaction as a fluid bed or a fixed bed, in gas phase reactions or liquid phase reactions.

Gas phase reaction in the sense of the present invention means that the reaction is carried out principally in a gas phase, in which a proportion of a liquid phase is in a range of from 0 % to 10 % by mass, preferably 0 % to 5 % by mass, more preferably 0 % to 2 % by mass and further preferably substantially 0 % by mass based on the total amount

of the raw materials. The carboxylic amide and the aliphatic alcohol which are the raw materials may be vaporized before they are supplied to a reactor or may be vaporized in the reactor. Furthermore, the carboxylic amide and the aliphatic alcohol may each be supplied separately to the reactor or may be supplied to the reactor as a mixture. Typically the reaction temperature is in a range of from 100 °C to 250 °C and the pressure is adjusted to 50 bar. The molar ratio of alcohol:carboxylic acid amide in the feed is 1:1 to 50:1, preferably the molar ratio is 2:1 to 30:1, more preferably the molar ratio is 5:1 to 30:1, most preferably the molar ratio is 7:1.

The carboxylic acid ester may be separated from the products by any known method, e.g. by distillation.

Liquid phase reaction in the sense of the present invention means that the reaction is carried out in a liquid phase. Any suitable solvent may be used or no solvent is used. In general, the reaction temperature depends on the boiling point of the alcohol and the reaction pressure. Therefore the temperature can be in the range of from 40 °C to 300 °C, preferably the temperature is in a range of from 100 °C to 250 °C. The reaction pressure can be in a range of from 1 bar to 100 bar, preferably the pressure is in a range of from 10 bar to 90 bar, more preferably the pressure is in a range of from 30 bar to 65 bar.

The molar ratio of alcohol:carboxylic acid amide in the feed is 1:1 to 50:1, preferably the molar ratio is 2:1 to 30:1, more preferably the molar ratio is 5:1 to 30:1, most preferably the molar ratio is 7:1.

The carboxylic acid ester may be separated from the products by any known method, e.g. by distillation.

**Methods of measurement**

Pore volume, referred to as $P_v(N_2)$, is determined in accordance with DIN 66134 ($N_2$-sorption according to Barret, Joyner, Halenda), DIN 66135 (gas adsorption), and ASTM D4222 ($N_2$-adsorption of catalysts and catalyst carriers).

Pore volume, referred to as $P_v(H_2O)$, is determined according to the following procedure:

The sample is weighed (10-25 g) and filled into a 250-mL beaker. 100 mL of distilled water is added. As soon as the formation of air bubbles ends, but not before the expiration of 15 minutes, the sample is separated from the water with a suitable sieve. The sample is evenly spread onto a filter paper (29x29cm, Fa. Schleicher & Schüll, Art. Nr. 0858) to remove water from the surface. The sample is weighed and the pore volume is determined by the following formula:

$$P_v(H_2O) = \frac{A-E}{E},$$

wherein
E = original sample weight [g],
A = resulting sample weight after water absorption [g]

The unit of the pore volume ($H_2O$ method) is ml/g because the density of water is 1 g/ml.

LOD (loss on drying) is determined according to the following procedure:

A porcelain crucible is dried in a laboratory-type drying cabinet with air circulation at 130 °C until constant weight on a precision balance (accuracy ± 0.1 mg). The crucible is cooled down to room temperature in an exsiccator and weighed. 5-10 g of the sample are weighed in and dried at 130 °C for approx. 4 h until constant weight. The sample is cooled down to room temperature in an exsiccator and the loss on drying is determined by the following formula

$$LOD\ [wt-\%] = \frac{E-A}{E} * 100,$$

wherein
E = original sample weight [g],
A = resulting sample weight after drying [g]
LOI (Loss on ignition) is determined according to the following procedure:

A porcelain crucible is heated in a laboratory-type drying cabinet with air circulation at 450 °C until constant weight on a precision balance (accuracy ± 0.1 mg). The crucible is cooled down to room temperature in an exsiccator and weighed. 5-10 g of the sample are weighed in and heated at 450 °C for approx. 2 h until constant weight. The sample is cooled down to room temperature in an exsiccator and the loss on drying is determined by the following formula

$$LOI\ [wt-\%] = \frac{E-A}{E} * 100,$$

wherein
E = original sample weight [g],
A = resulting sample weight after heating [g]

The pH value is determined according to the following procedure:

A pH meter is calibrated with buffer solutions (pH 7 and pH 4). 5.000 g of the sample are weighed into a 100-mL beaker and 50 mL deionized water are added. The slurry is gently stirred with a magnetic stirrer to ensure adequate suspension. The pH of the slurry is measured.

The isoelectic point, referred to as pI and sometimes abbreviated to IEP, is the pH at which the surface of a particular molecule or particle in a solution or suspension carries no net electrical charge. The IEP is a characteristic value for a molecule or particle and an important parameter to evaluate the stability of a suspension of particles.

The isoelectic point is determined in accordance with ISO 13099-1 (*Collidal systems - methods for zeta-potential determination*).

The content of Na and Cl is measured by flame atomic absorption spectrometry (F-AAS) according to the following procedure:

Approximately 250 mg of the sample are weighed accurately to 0.1 mg into a platinum bowl. 4 mL of a 1:1 diluted sulphuric acid and 10 mL of concentrated hydrofluoric acid are added. The sample material is digested by heating the bowl on a hot plate (approx. 325 °C) until fumes of the acid are emitted. Then 2 mL of 1:1 diluted sulphuric acid are added and the mixture is heated to dissolve the residue. The sample solution is transferred into a volumetric sample tube and filled up to 50 mL with ultrapure water.

The sample solution is measured using flame atomic absorption spectrometry (F-AAS) with air acetylene flame according to DIN 51401. Calibration solutions are prepared from stock solution, traceable to NIST-based reference material. Corresponding chemical blanks are measured together with sample solutions and subtracted if necessary.

X-Ray Powder Diffraction is measured by X'Pert MPD Pro diffractometer from PANalytical using the following parameters:

| | |
|---|---|
| X-ray tube: | LFF-Cu X-ray tube, Cu K$\alpha$, $\lambda$=0.1542 nm |
| Generator settings: | 40 mA, 40 KV |
| Detector: | X'Celerator |
| Rotation: | Yes / 1 Rev./s |
| 2-Theta range: | 5°-100° |
| Step (°2$\Theta$) | 0.017° |
| Time per step: | 40 s |

In addition, a shorter 2Theta range between 20° and 40° with longer measurement time of 160 s/step was recorded for better detection sensitivity of the monoclinic crystalline phase.

The bulk density is determined according to the following procedure:

An empty 250-ml measuring cylinder is placed onto a scale and the scale is adjusted to zero. The sample is filled into this cylinder until the 250-ml marking is reached. The weight is logged. The bulk density is calculated according to the following formula:

$$bd = \frac{E \cdot (100 - W)}{V \cdot 100},$$

wherein
bd = bulk density,
E = weighted sample [g],
V = sample volume [ml],
W = residual moisture content [wt-%] = LOD.

BET surface area is determined in accordance with DIN 66131 and DIN ISO 9277.

Abrasion is determined in accordance with ASTM D4058-96.

Particle size is determined by laser diffraction with a Beckman Coulter LS 13320, Universal Liquid Modul (Software "Beckman Coulter LS 13 320 Version 6.01").

The zirconium hydroxide is transformed into granules with an intensive mixer of the type R from Maschinenfabrik Gustav Eirich GmbH & Co KG. on the basis of a procedure disclosed in Keramische Zeitschrift 2003, 55, 598-604 and 681-684. The typical mixing speed is 3000 rpm. Alternatively, a rotating plate from Maschinenfabrik Gustav Eirich GmbH & Co KG. is used with a rotation speed of 1-4 m/sec.

The granules are sieved by using a mesh sieving machine, wherein the meshes differ in the size of the slots. During sieving the granules are graded to fractions with a defined particle size, whereby smaller and larger particles are separated out. Mesh 1 provides particles with a particle size in the range of from 1 mm to 10 mm, mesh 2 provides particles with a particle size in the range of from 0.8 mm to 5 mm, mesh 3 provides particles with a particle size in the range of from 0.8 mm to 2.5 mm, and mesh 4 provides particles with a particle size in the range of from 1.0 mm to 2.8 mm.

## Example 1 - method a

The pore volume of 1215 g zirconium hydroxide particles, XZO 1501/09 from MEL Chemicals, is determined to 850 mL. 136 g $Y(NO_3)_3x6H_2O$ are dissolved in 263 g $H_2O$, resulting in a solution of 340 mL, which corresponds to 40 % of the total pore volume. 380 g $H_2O$ are added to obtain a solution with a total volume of 680 mL, which corresponds to 80 % of the total pore volume. The zirconium hydroxide material is impregnated in an Eirich intensive mixer type R by spraying the yttrium nitrate solution under moderate stirring conditions (215 rpm) onto the zirconium hydroxide for a period of 10 minutes. Subsequently, the wet powder is transformed into granules with a particle size mainly in the range of from 0.1 mm to 10 mm under stirring conditions (3000 rpm) for 1 hour. The material is sieved through a mesh sieving machine with mesh 2 to obtain granules with a particle size in the range of from 0.8 mm to 5 mm. Afterwards, the granules are dried at 120 °C for 2 hours and then calcined at 450 °C for 2 hours. The granules are sieved again through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <65 ppm (F-AAS). The crystalline phase analysis exhibits a composition of 10 % monoclinic and 90 % tetragonal phase. Further properties of the mixed oxide are shown in table 3.

## Example 1 - method b

The pore volume of 1215 g zirconium hydroxide particles, XZO 1501/09 from MEL Chemicals, is determined to 850 mL. 136 g $Y(NO_3)_3x6H_2O$ are dissolved in 263 g $H_2O$, resulting in a solution of 340 mL, which corresponds to 40 % of the total pore volume. The zirconium hydroxide material is impregnated in an Eirich intensive mixer type R by spraying the yttrium nitrate solution under moderate stirring conditions (215 rpm) onto the zirconium hydroxide for a period of 10 minutes. Afterwards, 380 g water are added to the powder to have a total water volume that corresponds to 80 % of the total pore volume of the zirconium hydroxide material. Subsequently, the wet powder is transformed into granules with a particle size mainly in the range of from 0.1 mm to 10 mm under stirring conditions (3000 rpm) for 1 hour. The material is sieved through a mesh sieving machine with mesh 2 to obtain granules with a particle size in the range of from 0.8 mm to 5 mm. The fines (particle size < 0.8 mm) are collected for examples 2 and 3. Afterwards, the granules are dried at 120 °C for 2 hours and then calcined at 450 °C for 2 hours. The granules are sieved again through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <70 ppm (F-AAS). The crystalline phase analysis exhibits a composition of 8 % monoclinic and 92 % tetragonal phase. Further properties of the mixed oxide are shown in table 3.

## Example 2

108.8 g $Y(NO_3)_3x6H_2O$ are dissolved in 210.4 g $H_2O$, resulting in a solution of 272 mL, which corresponds to 40 % of the total pore volume. 380 g $H_2O$ are added to obtain a solution with a total volume of 544 mL, which corresponds to 80 % of the total pore volume.

972 g zirconium hydroxide with a total pore volume of 680 mL, purchased from MEL Chemicals (XZO 1501/09), are impregnated in an Eirich intensive mixer type R by spraying the yttrium nitrate solution under moderate stirring conditions (215 rpm) onto the zirconium hydroxide for a period of 10 minutes. After mixing for 5 minutes, 243 g zirconium hydroxide wet fines (particle size < 800 $\mu$m) from example 1 method a (waste after first sieving step) are added to the mixer and let run for 1 hour. The wet zirconium hydroxide fines from example 1 method a are characterized by pores filled in with yttrium nitrate solution (4 wt% on oxide basis). The powder is transformed into granules with a particle size mainly in the range of from 0.1 mm to 10 mm under stirring conditions (3000 rpm) for 1 hour. The material is sieved

through a mesh sieving machine with mesh 2 to obtain granules with a particle size in the range of from 0.8 mm to 5 mm. The granules are dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <115 ppm (F-AAS). The crystalline phase analysis exhibits a composition of 5 % monoclinic and 95 % tetragonal phase. Further properties of the mixed oxide are shown in table 3.

## Example 3

Mixture A: 108.8 g $Y(NO_3)_3x6H_2O$ are dissolved in 210.4 g $H_2O$, resulting in a solution of 272 mL, which corresponds to 40 % of the total pore volume. 380 g $H_2O$ are added to obtain a solution with a total volume of 544 mL, which corresponds to 80 % of the total pore volume.

Mixture B: 972 g zirconium hydroxide with a total pore volume of 680 mL, purchased from MEL Chemicals (XZO 1501/09), are impregnated by adding the yttrium nitrate solution to the hydroxide material using a mixer with high shear energy.

Mixture C: 300 g of wet zirconium hydroxide coarse particles from example 1 method a (particle size > 2.8 mm, waste after first sieving step) are sized down using a Nebulasizer type NS-60 from NARA Machinery Co., Ltd.. The wet zirconium hydroxide particles are homogeneously sized down to particle sizes lower than 1000 μm. The wet zirconium hydroxide coarse particles from example 1 method a are characterized by pores filled in with yttrium nitrate solution (4 wt% on oxide basis).

After mixing A and B for 10 minutes, 243 g zirconium hydroxide wet fines of mixture C (particle size < 800 μm) are added to the mixer and let run for 1 hour. The powder is transformed into granules with a particle size mainly in the range of from 0.1 mm to 10 mm. The material is sieved through a mesh sieving machine with mesh 4 to obtain granules with a particle size in the range of from 1.0 mm to 2.8 mm. Afterwards, the granules are dried at 120 °C for 2 hours and then calcined at 450 °C for 2 hours. The granules are sieved through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <95 ppm (F-AAS). The crystalline phase analysis exhibits a composition of < 3 % monoclinic and > 97 % tetragonal phase. Further properties of the mixed oxide are shown in table 3.

## Example 4

The pore volume of 1215 g zirconium hydroxide manufactured by MEL Chemicals (XZO 1501/09) is determined to 850 mL. 136 g $Y(NO_3)_3x6H_2O$ are dissolved in 263 g $H_2O$, resulting in a solution of 340 mL, which corresponds to 40 % of the total pore volume. 380 g $H_2O$ are added to obtain a solution with a total volume of 680 mL, which corresponds to 80 % of the total pore volume.

Thereafter, the zirconium hydroxide material is impregnated in an Eirich intensive mixer type R by spraying the yttrium nitrate solution under moderate stirring conditions (215 rpm) onto the zirconium hydroxide. The solution is added step wise over a period of 10 minutes. The impregnated powder is transformed into micro-granules with a size lower than 1 mm under stirring conditions (1.5 m/sec) for 45 min. The granules are transferred to a rotating plate (3.5 m/sec, 30 °C open air). The granules are agglomerating and drying at the same time and particles with a particle size mainly in the range of from 0.1 mm to 5 mm are produced. The material is sieved with mesh 2 to obtain granules with a particle size in the range of from 0.8 mm to 5 mm, and is dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium is <120 ppm (F-AAS). The crystalline phase analysis exhibits a composition of 7 % monoclinic and 93 % tetragonal phase. Further properties of the mixed oxide are shown in table 3.

## Example 5

Water (700 mL) is heated to 50 °C and held at this temperature during the precipitation procedure. The pH is adjusted to 10.5 with NaOH solution (1047 g of 20 wt-% solution) and is held constantly at this value during the precipitation

procedure.

Solution A is prepared by dissolving 61.5 g of yttrium nitrate in 100 mL of water. To this solution 1350 g of a zirconium nitrate solution, corresponding to 20 wt-% $ZrO_2$ in water, are added. Water is added to a total volume of 1500 mL.

The precipitation procedure is a continuous precipitation wherein solution A and 1047 g of NaOH solution (20 wt-% NaOH) are added continuously under constant stirring at a temperature of 50 °C over a period of 3 hours to maintain a constant pH of 10.5. A white precipitate is formed.

After 3 hours the precipitate is filtered and washed with water (4 liters) containing 50 g/l $NH_4NO_3$ in order to remove sodium. An additional washing step is performed using deionized water (2 liters). Afterwards the material is dried at 120 °C for 6 hours.

420 g of the dried precipitate are mixed with 275 ml of water, corresponding to 80 % of the total pore volume of 0.65 ml/g, using an Eirich intensive mixer type R. Under stirring conditions (3000 rpm) for 1 hour the powder is transformed into granules with a particle size mainly in the range of from 0.1 mm to 10 mm. The material is sieved with mesh 2 to obtain granules with a particle size in the range of from 0.8 mm to 5 mm and dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <120 ppm (F-AAS). The crystalline phase analysis exhibits a composition of < 3 % monoclinic and > 97 % tetragonal phase. Further properties of the mixed oxide are shown in table 3.

## Example 6

Water (700 mL) is heated to 50 °C and held at this temperature during the precipitation procedure. The pH is adjusted to 10 with $NH_4OH$ solution (4300 g of a 25 wt-% $NH_4OH$ solution) and is held constantly at this value during the precipitation procedure.

Solution A is prepared by dissolving 61.5 g of yttrium nitrate in 100 mL of water. To this solution 1350 g of a zirconium nitrate solution, corresponding to 20 wt-% $ZrO_2$ in water, are added. Water is added to a total volume of 1500 mL.

The precipitation procedure is a continuous precipitation wherein solution A and 4300 g of $NH_4OH$ solution (25 wt-% $NH_4OH$) are added continuously under constant stirring at a temperature of 50 °C over a period of 3 hours to maintain a constant pH of 10. A white precipitate is formed.

After 3 hours the precipitate is filtered and washed with water (2 liters). After drying at 120 °C for 6 hours the material is milled.

420 g of the dried precipitate are mixed with 275 ml of water, corresponding to 80 % of the total pore volume of 0.65 ml/g, using an Eirich intensive mixer type R. Under stirring conditions (3000 rpm) for 1 hour the powder is transformed into granules with a particle size mainly in the range of from 0.1 mm to 10 mm. The material is sieved with mesh 2 to obtain granules with a particle size in the range of from 0.8 mm to 5 mm and dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <25 ppm (F-AAS). The crystalline phase analysis exhibits a composition of < 3 % monoclinic and > 97 % tetragonal phase. Further properties of the mixed oxide are shown in table 3.

## Example 7

Water (700 mL) is heated to 50 °C and held at this temperature during the precipitation procedure. The pH is adjusted to 8.9 with $NH_4OH$ solution (450 g of a 25 wt-% $NH_4OH$ solution) and is held constantly at this value during the precipitation procedure.

Solution A is prepared by adding water to 950 g of a zirconium nitrate solution, corresponding to 20 wt-% of $ZrO_2$ in water, to a total volume of 1500 mL.

The precipitation procedure is a continuous precipitation wherein solution A and 450 g of $NH_4OH$ solution (25 wt-% $NH_4OH$) are added continuously under constant stirring at a temperature of 50 °C over a period of 3 hours to maintain a constant pH of 8.9. A white precipitate is formed.

After 3 hours the precipitate is filtered and washed with water (2 liters). The resulting material is dried at 120 °C for 6 hours to yield zirconium hydroxide. The pore volume is determined by using the pore volume $H_2O$ method to 0.65 ml/g.

100 g of the so-prepared zirconium hydroxide are mixed with a solution containing yttrium nitrate (11.7 g $Y(NO_3)_3 {*} 6H_2O$ in 52 mL water) using a high shear energy mixer. The total volume of the solution used is up to 80 % of the total pore volume of the zirconium hydroxide powder. Under mixing conditions (3000 rpm) for 1 hour the powder is transformed into granules with a particle size mainly in the range of from 0.1 mm to 10 mm. The material is sieved with

mesh 2 to obtain granules with a particle size in the range of from 0.8 mm to 5 mm and dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 4 wt-% $Y_2O_3$, which corresponds to a molar ratio Zr:Y = 98:2. The sodium content is <50 ppm (F-AAS). The crystalline phase analysis exhibits a composition of 13 % monoclinic and 87 % tetragonal phase. Further properties of the mixed oxide are shown in table 3.

**Comparative example 1 (CE1)**

Water (700 ml) is heated to 50 °C and held at this temperature during the precipitation procedure. The pH is adjusted to 10.5 with NaOH solution (1047 g of a 20 wt-% solution) and is held constantly at this value during the precipitation procedure.

Solution A is prepared by dissolving 61.5 g of yttrium nitrate in 100 ml of water. To this solution 1350 g of a zirconium nitrate solution, corresponding to 20 wt-% $ZrO_2$ in water, are added. Water is added to a total volume of 1500 mL.

The precipitation procedure is a continuous precipitation wherein solution A and 1047 g of NaOH solution (20 wt-% NaOH) are added continuously under constant stirring at a temperature of 50 °C over a period of 3 hours to maintain a constant pH of 10.5. A white precipitate is formed.

After 3 hours the precipitate is divided in 3 parts and in order to remove sodium is filtered and washed with water. The 3 portions are washed separately by using different amounts of water, respectively, (i) 5 liters, (ii) 20 liters and (iii) 30 liters. Afterwards the materials were dried at 120 °C for 6 hours.

Each of the dried precipitates is mixed with an amount of water corresponding to 80% of the total pore volume of 0.6 ml/g using an Eirich intensive mixer type R. Under stirring conditions (3000 rpm) for 1 hour the powder is transformed into granules with a particle size mainly in the range of from 0.1 mm to 10 mm. The material is sieved with mesh 2 to obtain granules with a particle size in the range of from 0.8 mm and 5 mm and dried at 120 °C for 2 hours and then calcined at 450 °C for 2 hours. The granules are passed through a mesh sieving machine with mesh 3 to obtain granules with a particle size in the range of from 0.8 mm to 2.5 mm.

The mixed oxide contains 6 wt-% $Y_2O_3$, which corresponds to a molar ratio Zr:Y = 97:3. The sodium content, determined as $Na_2O$, because F-AAS is not applicable, is, respectively, (i) 6.6 wt%, (ii) 2.0 wt% and (iii) 1.4 wt%.

The crystalline phase analysis exhibits the following composition:

(i) 68 % tetragonal phase, 32 % monoclinic phase,

(ii) 72 % tetragonal phase, 28 % monoclinic phase,

(iii) 75 % tetragonal phase, 25 % monoclinic phase.

Further properties of the mixed oxides are shown in table 3.

**Comparative example 2 (CE2)**

For CE2 a catalyst was produced according to example 42 of EP2415750.

The obtained mixed oxide contains 4 wt-% $Y_2O_3$, which corresponds to a molar ratio Zr:Y = 98:2. The sodium content is < 180 ppm (F-AAS). The crystalline phase analysis exhibits a composition of 17% monoclinic and 83 % tetragonal phase. Further properties of the mixed oxide are shown in table 3.

**Comparative example 3 (CE3)**

For CE3 compound SZ6157 is purchased from Saint-Gobain NorPro and applied as received. This material exhibits the following characteristics according to the product specification:

Surface area: 115 $m^2/g$,
Median pore diameter: 5/150 nm
Total pore volume (Hg): 0.33 $cm^3/g$
Packing density: 1250 $kg/m^3$
Phase: tetragonal
Purity: 8 % $Y_2O_3$
Shape: extrudates/pellets (3 mm)

Table 3: Properties of mixed oxide granules (particle size 0.8 - 2.5 mm).

| Entry | Zr/Y molar ratio | Tetragonal phase [%] | BET [$m^2/g$] | Abrasion [wt-%] | Bulk density [g/L] | Pv($H_2O$) [ml/g] | Na content |
|---|---|---|---|---|---|---|---|
| 1a | 98:2 | 90 | 125 | 2.5 | 1100 | 0.38 | < 65 ppm |
| 1b | 98:2 | 92 | 145 | 1.3 | 980 | 0.42 | < 70 ppm |
| 2 | 98:2 | 95 | 150 | 1.9 | 1050 | 0.42 | < 115 ppm |
| 3 | 98:2 | > 97 | 135 | 1.5 | 1020 | 0.40 | < 95 ppm |
| 4 | 98:2 | 93 | 120 | 2.5 | 1150 | 0.36 | < 120 ppm |
| 5 | 98:2 | > 97 | 120 | 1.0 | 1020 | 0.38 | < 120 ppm |
| 6 | 98:2 | > 97 | | 1.3 | 980 | 0.40 | < 25 ppm |
| 7 | 98:2 | 87 | 130 | 1.1 | 1050 | 0.38 | < 50 ppm |
| CE1 | (i) 97:3 | (i) 68 | (i) 82 | (i) 1.3 | (i) 950 | (i) 0.36 | (i) 6.6 wt-% |
| | (ii) 97:3 | (ii) 72 | (ii) 90 | (ii) 1.1 | (ii) 950 | (ii) 0.36 | (ii) 2.0 wt-% |
| | (iii) 97:3 | (iii) 75 | (iii) 96 | (iii) 1.3 | (iii) 950 | (iii) 0.36 | (iii) 1.4 wt-% |
| CE2 | 98:2 | 83 | 140 | 3 | 1100 | 0.38 | < 180 ppm |
| CE3 | 92:8 | n.d. | 180 | n.d. | n.d. | 0.33 | 180 ppm |

Alcoholysis reaction

The afore-mentioned catalysts were tested in a methanolysis reaction of 2-HIBA in an electrically heated fixed bed reactor (diameter = 10 mm) made from stainless steel. 16 g of catalyst were applied and fixed with inert glass wool. A feed consisting of a mixture of methanol and 2-hydroxyisobutyric acid (2-HIBA) in a molar ratio of 7:1 (purity 98.5 %) is passed from below through the catalyst bed with a feed rate of 2 mL/min at a temperature in a range of from 200 °C to 220 °C. The pressure is adjusted to 60 bar with a pressure retention valve. A part of the product stream is separated and cooled with solid carbon dioxide. The sample is analysed by gaschromatography using a temperature program between 40 °C and 230 °C with a heating rate of 15 K/min. The results are shown in table 4.

| Component | Description |
|---|---|
| GC | CP-3800 Gas-phase chromatograph |
| Injector | |
| Front channel | 1079 PTV |
| Back channel | 1177 Split/Splitless |
| Column | |
| Front channel | DB WAXERT (Agilent) |

(continued)

| Component | Description |
|---|---|
| | Length 30 m<br>ID 0.53 mm<br>Film 1.5 μm<br>+ precolumn |
| Back channel | AT-WAX (Alltech)<br>Length 30 m<br>ID 0.53 mm<br>Film 1.2 μm |
| Detector | |
| Front channel | WLD (Thermal conductivity detector) |
| Back channel | FID (Flame ionisation detector) |

Table 4: Reaction conditions for methanolysis of 2-HIBA.

| Example | Temp [°C] | Pressure [bar] | Residence time of catalyst [g/mL*min] | Conversion rate at 72 h [%] | Selectivity at 72 h [%] |
|---|---|---|---|---|---|
| 1a | 200 | 60 | 8 | 30.8 | 98.4 |
| 1 b | 200 | 60 | 8 | 29.6 | 96.9 |
| 2 | 200 | 60 | 8 | 29.8 | 98.5 |
| 3 | 200 | 60 | 8 | 29.3 | 97.1 |
| 4 | 200 | 60 | 8 | 29.2 | 98.9 |
| 5 | 200 | 60 | 8 | 26.5 | 94.6 |
| 6 | 200 | 60 | 8 | 28.9 | 95.3 |
| 7 | 200 | 60 | 8 | 28.2 | 98.6 |
| CE1 | 200 | 60 | 8 | (i) 18.1 | (i) 88.9 |
| | | | | (ii) 20.4 | (ii) 90.5 |
| | | | | (iii) 22.5 | (iii) 92.5 |
| CE2 | 200 | 60 | 8 | 28.4 | 95.9 |
| CE3 | 200 | 60 | 8 | 21.2 | 99.3 |

The catalysts 1-7 show also a good performance in methanolysis reactions of 2-HIBA in the gas phase, wherein a conversion rate of up to 94 % and a selectivity of up to 97 % can be achieved.

## Claims

1. A mixed oxide containing zirconium and yttrium, wherein the tetragonal crystalline phase is present in an amount of from 85 % to 100 % and wherein the content of Na is in a range of from 20 ppm to 150 ppm.

2. The mixed oxide according to claim 1, wherein the molar ratio of Zr:Y, calculated as $ZrO_2$ and $Y_2O_3$, is in a range of from 90:10 to 98:2.

3. A process for the production of a mixed oxide according to claim 2, wherein the process comprises the following steps:

   a. providing zirconium hydroxide with a content of Na in a range of from 20 ppm to 150 ppm,

b. providing an aqueous solution containing at least one yttrium salt, wherein the volume of said solution corresponds to 40 % to 75 % of the pore volume $P_v(H_2O)$ of the Zirconium hydroxide, and wherein yttrium is present in said solution in such an amount that the mixed oxide exhibits 2 wt-% to 10 wt-% yttrium, calculated as $Y_2O_3$ and referring to the total weight of the mixed oxide,
c. contacting zirconium hydroxide with the solution obtained in step b),
d. adding water in an amount of from 0 % to 35 % of the pore volume $P_v(H_2O)$ of zirconium hydroxide, wherein the total amount of water added to the zirconium hydroxide is at most 75 % of the pore volume $P_v(H_2O)$ of the Zirconium hydroxide,
e. drying the zirconium hydroxide,
f. calcining the zirconium hydroxide to obtain the mixed oxide.

**4.** The process according to claim 3, wherein the zirconium hydroxide is further treated by moulding before or after drying.

**5.** A mixed oxide obtainable by a process according to one of claims 3 and 4.

**6.** A catalyst system comprising a mixed oxide according to claim 1 or claim 2 and a catalyst support.

**7.** Use of a mixed oxide according to claim 1, 2 or 5 as a catalyst or catalyst system.

**8.** Use according to claim 7 in alcoholysis reactions.

**9.** Use according to claim 8 in methanolysis reactions for the production of carboxylic acid esters.

**10.** A process for an alcoholysis reaction for the production of carboxylic acid esters, wherein a mixed oxide according to claim 1 or 2 is applied.

**11.** The process according to claim 10, wherein the process is a methanolysis reaction.

**12.** The process according to claim 11, wherein the methanolysis reaction is performed as gas phase reaction.

**13.** The process according to claim 11, wherein the methanolysis reaction is performed as liquid phase reaction.

**14.** The process according to anyone of claims 10-13, wherein the carboxylic acid ester is a hydroxyl carboxylic acid ester.

Europäisches Patentamt
European Patent Office
Office européen des brevets

# EUROPEAN SEARCH REPORT

Application Number
EP 14 19 5176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 415 750 A1 (MITSUBISHI GAS CHEMICAL CO [JP]) 8 February 2012 (2012-02-08)<br>* claims; examples 15,16,40-45 *<br>* paragraphs [0011] - [0018] * | 1-14 | INV.<br>C07C67/08<br>C01G25/02<br>B01J23/00<br>B01J23/10<br>B01J35/00<br>B01J37/02<br><br>ADD.<br>B01J35/02<br>B01J35/10<br>B01J37/03<br>B01J37/06<br>B01J37/08<br>B01J37/00 |
| X | MERCERA P D L ET AL: "STABILIZED TETRAGONAL ZIRCONIUM OXIDE AS A SUPPORT FOR CATALYSTS EVOLUTION OF THE TEXTURE AND STRUCTURE ON CALCINATION IN STATIC AIR", APPLIED CATALYSIS, AMSTERDAM, NL, vol. 78, no. 1, 29 October 1991 (1991-10-29), pages 79-96, XP001207970, ISSN: 0166-9834, DOI: 10.1016/0166-9834(91)80090-J<br>* abstract *<br>* page 80, line 6 - line 21 *<br>* page 81, line 33 - page 82, line 6 *<br>* Paragraph "Preparation of the samples" Paragraph "Chemical analysis" Paragraph "Conclusions" *<br>* table 2 * | 1,2,5-7 | |
| X | US 5 262 373 A (DURAND BERNARD [FR] ET AL) 16 November 1993 (1993-11-16)<br>* examples *<br>* column 3, line 38 - line 50 * | 1,2,5-7 | TECHNICAL FIELDS SEARCHED (IPC)<br>C07C<br>C01G<br>B01J |

-/--

The present search report has been drawn up for all claims

1

| Place of search | Date of completion of the search | Examiner |
|---|---|---|
| Munich | 29 May 2015 | Besselmann, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 5176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DELL'AGLI G ET AL: "Hydrothermal synthesis of ZrO2-Y2O3 solid solutions at low temperature", JOURNAL OF THE EUROPEAN CERAMIC SOCIETY, ELSEVIER SCIENCE PUBLISHERS, BARKING, ESSEX, GB, vol. 20, no. 2, February 2000 (2000-02), pages 139-145, XP004244436, ISSN: 0955-2219, DOI: 10.1016/S0955-2219(99)00151-X<br>* Paragraph 2. "Experimental procedure" *<br>* table 2 *<br>* abstract *<br>----- | 1,2,5,6 | |
| A | YAMAGUCHI T: "APPLICATION OF ZRO2 AS A CATALYST AND A CATALYST SUPPORT", CATALYSIS TODAY, AMSTERDAM, NL, vol. 20, 1994, pages 199-217, XP001039904, DOI: 10.1016/0920-5861(94)80003-0<br>* the whole document *<br>----- | 1-14 | TECHNICAL FIELDS SEARCHED (IPC) |
| A | KYOKO TAKAHASHI ET AL: "The esterification of carboxylic acid with alcohol over hydrous zirconium oxide.", BULLETIN OF THE CHEMICAL SOCIETY OF JAPAN, vol. 62, no. 7, 1989, pages 2353-2361, XP055191844, ISSN: 0009-2673, DOI: 10.1246/bcsj.62.2353<br>* the whole document *<br>----- | 1-14 | |

The present search report has been drawn up for all claims

1

| Place of search | Date of completion of the search | Examiner |
|---|---|---|
| Munich | 29 May 2015 | Besselmann, Sonja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 5176

This annex lists the patent family members relating to the patent documents cited in the above-mentioned European search report. The members are as contained in the European Patent Office EDP file on
The European Patent Office is in no way liable for these particulars which are merely given for the purpose of information.

29-05-2015

| Patent document cited in search report | Publication date | Patent family member(s) | Publication date |
| --- | --- | --- | --- |
| EP 2415750 A1 | 08-02-2012 | CN 102369178 A | 07-03-2012 |
| | | EP 2415750 A1 | 08-02-2012 |
| | | KR 20120004432 A | 12-01-2012 |
| | | RU 2011144499 A | 10-05-2013 |
| | | TW 201041845 A | 01-12-2010 |
| | | US 2012095253 A1 | 19-04-2012 |
| | | WO 2010113964 A1 | 07-10-2010 |
| US 5262373 A | 16-11-1993 | DE 69118078 D1 | 25-04-1996 |
| | | DE 69118078 T2 | 24-10-1996 |
| | | EP 0478768 A1 | 08-04-1992 |
| | | ES 2087294 T3 | 16-07-1996 |
| | | FR 2661169 A1 | 25-10-1991 |
| | | JP 3310666 B2 | 05-08-2002 |
| | | JP H05501102 A | 04-03-1993 |
| | | US 5262373 A | 16-11-1993 |
| | | WO 9116265 A1 | 31-10-1991 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2415750 A
- JP H06345692 B


### Non-patent literature cited in the description


- *Acta Cryst.,* 1967, vol. 22, 151-152
- *J. Appl. Cryst.,* 1969, vol. 2, 65-71
- *Keramische Zeitschrift,* 2003, vol. 55, 598-604, 681-684